(19) Europäisches Patentamt

European Patent Office

Office européen des brevets

(11) EP 0 526 602 B1

(12) FASCICULE DE BREVET EUROPEEN

(45) Date de publication et mention
de la délivrance du brevet:
**02.01.1997 Bulletin 1997/01**

(21) Numéro de dépôt: **92903775.2**

(22) Date de dépôt: **19.02.1992**

(51) Int Cl.[6]: **C12Q 1/00**, C12Q 1/54

(86) Numéro de dépôt international:
**PCT/CH92/00034**

(87) Numéro de publication internationale:
**WO 92/14836 (03.09.1992 Gazette 1992/23)**

(54) **CAPTEUR DE MESURE DE LA QUANTITE D'UN COMPOSANT EN SOLUTION**

Rezeptor zur Messung der Quantität eines Bestandteils in Lösung

SENSOR FOR MEASURING THE QUANTITY OF A DISSOLVED COMPONENT

(84) Etats contractants désignés:
**AT BE CH DE DK ES FR GB GR IT LI LU MC NL SE**

(30) Priorité: **21.02.1991 FR 9102200**

(43) Date de publication de la demande:
**10.02.1993 Bulletin 1993/06**

(73) Titulaire: **ASULAB S.A.**
**CH-2501 Bienne (CH)**

(72) Inventeurs:
- **GRÄTZEL, Michael**
  **CH-1025 St-Sulpice (CH)**
- **FRASER, David**
  **CH-1800 Vevey (CH)**
- **ZAKEERUDDIN, Shaik, Mohammed**
  **CH-1020 Renens (CH)**
- **RANDIN, Jean-Paul**
  **CH-2016 Cortaillod (CH)**
- **FRENKEL, Erik, Jan**
  **CH-2000 Neuchâtel (CH)**

(74) Mandataire: **Thérond, Gérard Raymond et al**
**I C B**
**Ingénieurs Conseils en Brevets SA**
**Rue des Sors 7**
**2074 Marin (CH)**

(56) Documents cités:
**EP-A- 0 096 288** **WO-A-85/05119**

- **ANGEWANDTE CHEMIE vol. 102, no. 1, 1990, WEINHEIM BRD pages 109 - 111; M.V. PISHKO ET AL: 'Directer Ektronenaustausch Zwischen Graphitelektroden und einem adsorbierten Komplex aus Glucose-Oxidase und einem Os-haltige Redoxpolymer'**
- **JOURNAL OF ELECTROANALYTICAL CHEMISTRY vol. 286, 1990, LAUSANNE CH pages 75 - 87; J.P. COLLIN et al**
- **ANALYTICAL CHEMISTRY vol. 54, 1982, WASHINGTON DC USA pages 1377 - 1383; J.M. JOHNSON ET AL: 'Potential-dependent enzymatic activity in an enzyme thin-layer cell'**
- **BIOCHEMISTRY vol. 24, no. 7, 26 Mars 1985, WASHINGTON DC USA pages 1579 - 1585; J.M. JOHNSON: 'redox activation of galactose oxidase: thin layer electrochemical study'**
- **PATENT ABSTRACTS OF JAPAN vol. 12, no. 96 (C-484)(2943) 29 Mars 1988**

## Description

La présente invention concerne un capteur de mesure de la quantité d'un composant en solution, destiné à être utilisé dans un dispositif de mesure ampérométrique de la concentration dudit composant dans la solution. Ce capteur permet notamment de doser le glucose.

De nombreux malades atteints de diabète, doivent mesurer fréquemment leur taux de glucose sanguin ou glycémie. S'ils détectent un état d'hyperglycémie, ils doivent immédiatement prendre des médicaments régulant leur taux de glucose. Afin de faciliter la vie courante de ces malades, de nombreux dispositifs de mesure du glucose miniaturisés et utilisables par un néophyte, sont apparus sur le marché.

Par ailleurs, on envisage de réaliser des implantations de pompes à insuline chez les diabétiques. Ces pompes à insuline doivent être munies de dispositifs de mesure du glucose également implantables et qui, en fonction de la glycémie mesurée, donnent une information à la pompe pour la mise en marche éventuelle de celle-ci.

La plupart de ces dispositifs de mesure de la glycémie utilisent une enzyme spécifique du glucose, la glucose oxydase (GOD).

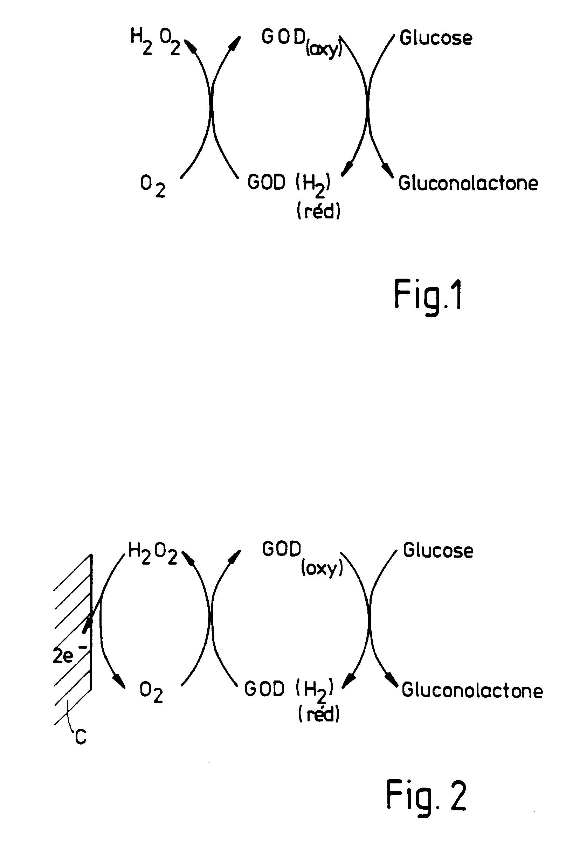

Comme illustrée à la figure 1 annexée, la GOD est une flavoprotéine, (obtenue par exemple à partir de moisissures) qui catalyse l'oxydation du glucose, ici par exemple le glucose sanguin, en gluconolactone, avec formation de peroxyde d'hydrogène $H_2O_2$, à partir de l'oxygène moléculaire $O_2$ présent dans la solution à tester, ici dans le sang.

Cette enzyme (la GOD) et l'oxygène, ont donc été fréquemment utilisés dans des dispositifs de mesure du glucose dans lesquels l'oxydation du glucose était détectée par un transducteur électrique ou optique.

De même, cette enzyme (la GOD) et l'oxygène ont souvent été utilisés dans des dispositifs dits ampérométriques et leur emploi est décrit dans la littérature.

Ces dispositifs dits "ampèrométriques" comprennent d'une part, un appareil de mesure muni d'au moins deux contacts électriques reliés à un ampèremètre et à des moyens d'affichage et d'autre part, un capteur éventuellement jetable, pouvant être relié à ces deux contacts électriques. Ce capteur comprend au moins deux électrodes, l'une de référence et l'autre de mesure. L'électrode de mesure comprend un conducteur métallique recouvert d'une enzyme spécifique du produit que l'on cherche à détecter.

La figure 2 annexée illustre les réactions chimiques survenant au niveau de cette électrode de mesure. Lorsque l'on dépose la solution à tester sur l'électrode de mesure, le produit à tester (ici le glucose) réagit avec l'enzyme (ici la GOD oxydée) se trouvant sur l'électrode, pour former du gluconolactone, tandis que la GOD passe à l'état réduit (GOD $(H_2)_{(réd)}$). Cette GOD réduite réagit alors avec l'oxygène $O_2$ qui passe à l'état réduit $H_2O_2$ et qui transfère alors deux électrons e⁻ vers le conducteur électrique C dont le potentiel est fixe et se situe aux alentours de 650 mV. Le fait que l'on soit obliger de travailler à des potentiels élevés entraîne des problèmes supplémentaires d'interférences. L'oxygène a donc un rôle de médiateur, puisqu'il permet le transfert d'électrons. Ce transfert d'électrons, proportionnel à la quantité de glucose présente dans la solution à tester, est alors mesuré par l'ampèremètre et la quantité du glucose présente dans la solution est affichée par les moyens d'affichage de l'appareil de mesure.

Des recherches complémentaires ont montré que des dispositifs ampérométriques utilisant des médiateurs non physiologiques, organiques, inorganiques ou organométalliques pouvaient supplanter les dispositifs utilisant l'oxygène comme médiateur. En effet, comme on peut le constater sur la figure 2, les dispositifs utilisant l'oxygène comme médiateur ne peuvent pas être utilisés dans des solutions où la concentration stoechiométrique en oxygène est inférieure à la concentration du composant que l'on désire mesurer. Car sinon, dans ce cas, tandis que la totalité du composant à mesurer a la possibilité de réagir avec l'enzyme oxydée pour former l'enzyme réduite, seule une partie de la quantité totale d'enzyme réduite peut réagir avec l'oxygène présent, proportionnellement à cette quantité d'oxygène. Le reste de l'enzyme réduite ne peut pas réagir et la quantité d'électrons transmise au conducteur C est inférieure à ce qu'elle devrait être.

En conséquence, lorsqu'on utilise ce type de dispositif, on est soit limité par les concentrations respectives de l'oxygène et du composant à mesurer, soit obligé d'utiliser une membrane pour limiter la diffusion dudit composant. Ceci explique pourquoi on a cherché à réaliser des dispositifs ampérométriques utilisant un médiateur particulier, remplaçant l'oxygène.

De très nombreux médiateurs ont été cités dans la littérature, tels que les ferrocènes monomères (Cass, A.E.G. et ses collaborateurs (1984), Anal. Chem. 56, 667-671; Degani, Y. et Heller, A. (1987), J. Phys, Chem. 91, 1285-1289), les ferrocènes greffés sur un polymère (Foulds, N.C. et Lowe, C.R. (1988) Anal. Chem. 60, 2473-2478), les sels conducteurs de transfert de charge (Albery, W.J. Bartlett, P.N. et Craston, D.H. (1985) J. Electroanal. Chem. Interfacial. Electrochem, 194, 223-235), les nickels cyclames (Taniguchi, I., Matsushita, K., Okamoto, M., Collin, J-P et Sauvage, J-P (1990) J. Electroanal. Chem. Interfacial. Electrochem. 280, 221-226) des composés organiques tels que les quinones et les benzoquinones (Kulys, J.J., et Cénas, N.K. (1983) Biochim. Biophys. Acta 744, 57), et des complexes de divers métaux de transition, sans mention de l'osmium, avec divers ligands, dont la bipyridine non substituée (EP 0 096 288).

Il convient toutefois de signaler que les travaux de Pishko M.V. et al (Angew. Chem. _102_ (1990), 109-111) font état d'un terpolymère, utilisé comme médiateur de transfert d'électrons de l'enzyme GOD, dans lequel un des monomères est constitué par un vinyl pyridine N-substitué par un complexe d'osmium avec deux ligands bipyridine non substitués (-Os(bpy)2Cl).

Du fait des travaux importants de Hill et ses collaborateurs, par exemple Frew. J.E., et Hill, H.A.O. (1987) (Phil. Trans. R. Soc. Lond. B316, 95-106)), la famille des composés du ferrocène a été largement instituée et utilisée comme médiateur pour la GOD et d'autres flavoprotéines. En conséquence, on connaît un capteur actuellement commercialisé utilisant comme médiateur, un membre de la famille des composés du ferrocène.

Malheureusement, les médiateurs existant actuellement ont rarement les propriétés idéales requises, à savoir, un potentiel électrochimique adapté à l'enzyme choisie, une solubilité adéquate, une bonne stabilité chimique à la lumière, à la température et au pH et une interaction rapide avec l'enzyme choisie.

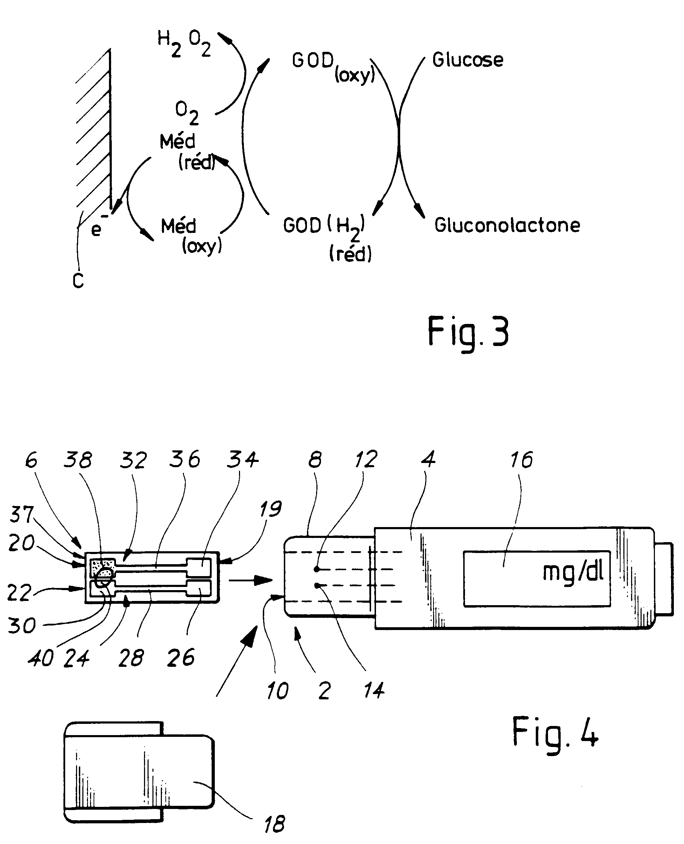

De plus, l'oxygène présent éventuellement dans les solutions à tester entre en compétition avec certains médiateurs selon le schéma de la figure 3 annexée. En effet, tandis que le médiateur Méd présent sur le conducteur C continue de réagir avec certaines molécules de GOD réduite, il est possible qu'une certaine quantité de l'oxygène $O_2$ éventuellement présent réagisse également avec d'autres molécules de GOD réduite en formant du $H_2O_2$, comme vu précédemment à la figure 2. Lorsque l'on effectue des mesures avec un faible potentiel entre l'électrode de mesure et l'électrode de référence, le $H_2O_2$ piège les électrons provenant de la réaction entre le GOD et l'oxygène et ces électrons ne passent plus vers l'électrode. Comme la quantité d'oxygène en solution peut varier, la quantité d'électrons piégés varie également. En conséquence, il n'existe alors plus de proportionnalité entre la quantité d'électrons passant vers l'électrode et la quantité de glucose se trouvant dans la solution à tester. Dans ces conditions, ces capteurs ne donnent donc pas de résultats fiables.

L'invention a pour objet de remédier aux inconvénients précédemment cités.

A cet effet, l'invention concerne un capteur de mesure de la quantité d'un composant en solution, comprenant :

- au moins une électrode de mesure et une électrode de référence, isolées électriquement l'une de l'autre et destinées à venir en contact avec ladite solution, lesdites électrodes comprenant respectivement des contacts électriques adaptés pour être branchés sur un dispositif d'exploitation du signal fourni par ledit capteur,
- l'électrode de mesure comprenant au moins un collecteur de courant, relié électriquement à l'un desdits contacts électriques et recouvert d'un mélange comprenant au moins une enzyme d'oxydo-réduction spécifique dudit composant et au moins un médiateur transférant les électrons entre ladite enzyme et ledit collecteur de courant.

Selon les caractéristiques de l'invention, le médiateur est choisi parmi les complexes osmium avec des ligands bipyridine substitués par au moins un groupe donneur d'électrons choisi parmi le groupe OH, un groupe alkoxy, un groupe aryloxy, ou un groupe amine primaire secondaire ou tertiaire.

Grâce aux caractéristiques du capteur selon l'invention et notamment grâce aux nouveaux médiateurs utilisés, on obtient une famille de capteurs présentant une large gamme de faibles potentiels d'oxydo-réduction, restant stables à l'air et fournissant une réponse plus rapide que les autres capteurs de l'art antérieur.

L'invention sera mieux comprise à la lecture de la description suivante des modes préférentiels de réalisation de l'invention, donnés à titre indicatif mais non limitatif, cette description étant faite en liaison avec les dessins joints dans lesquels :

- la figure 1 illustre la dégradation du glucose en présence de glucose oxydase GOD,
- les figures 2 et 3 sont des schémas illustrant les diverses réactions chimiques survenant au niveau des capteurs,
- la figure 4 est une vue de dessus d'un appareil de mesure muni d'un capteur selon l'invention,
- la figure 5 représente les courbes de voltamétrie cyclique du complexe tris (4,4' - diméthoxy - 2,2' - bipyridine) d'osmium en l'absence de GOD et de glucose, à différentes vitesses de balayage,
- la figure 6 représente sensiblement les mêmes courbes que la figure 5, mais en présence de GOD et de glucose,
- la figure 7 représente trois courbes illustrant la variation de la densité de courant obtenue après 30 secondes ($D_{30}$) en fonction de la concentration en glucose dans une solution physiologique, pour des mesures effectuées avec trois types de capteurs selon l'invention, dans lesquels la quantité de poudre de carbone varie,
- la figure 8 représente la pente et l'ordonnée à l'origine des courbes de la figure 7, en fonction de la quantité de poudre de carbone,
- la figure 9 représente trois courbes illustrant la variation de la densité de courant obtenue après 30 secondes ($D_{30}$) en fonction de la concentration en glucose dans une solution physiologique, pour des mesures effectuées avec trois types de capteurs selon l'invention, dans lesquels la quantité de glucose oxydase varie,
- la figure 10 représente la pente et l'ordonnée à l'origine des courbes de la figure 9, en fonction de la quantité de glucose oxydase,
- la figure 11 représente trois courbes illustrant la variation de la densité de courant obtenue après 30 secondes ($D_{30}$) en fonction de la concentration en glucose dans une solution physiologique, pour des mesures effectuées avec trois types de capteurs selon l'invention, dans lesquels la quantité de médiateur varie,

- la figure 12 représente la pente et l'ordonnée à l'origine des courbes de la figure 11, en fonction de la quantité de médiateur,
- la figure 13 représente des mesures de la densité de courant obtenue en fonction de la concentration en glucose, ces mesures étant effectuées dans le sang et dans un tampon phosphate avec des capteurs de glucose munis respectivement d'un des deux médiateurs préférés selon l'invention,
- la figure 14 représente des mesures de la densité de courant obtenue en fonction de la concentration en glucose, ces mesures étant effectuées avec des capteurs selon l'invention dans des échantillons de sang présentant des hématocrites différents,
- les figures 15 et 16 représentent des mesures de la densité de courant obtenue en fonction de la concentration en glucose, ces mesures étant effectuées avec des capteurs selon l'invention dans des échantillons de solution physiologique présentant respectivement diverses concentrations d'acétaminophénol et d'acide ascorbique.

Comme illustré en figure 4, l'appareil de mesure 2 de la quantité d'un composant donné dans une solution, comprend un capteur 6 selon l'invention et un dispositif d'exploitation 4 du signal fourni par ledit capteur. Ce dispositif 4 est connu en soi et présente la forme générale d'un stylo. Il est bien évident que cette forme n'est pas limitative de l'invention.

Ce stylo 4 comprend à l'une de ses extrémités référencée 8, une cavité 10 dans laquelle sont logés deux premiers contacts électriques 12, 14 connectés électriquement à un ampèremètre (non représenté). Cet ampèremètre est lui-même relié à un affichage 16 indiquant la concentration du composant recherché dans une solution donnée. Cette concentration est affichée, par exemple en mg/dl ou en mmol/l. Le stylo 4 comprend en outre un bouchon 18 qui vient coiffer son extrémité 8 et protéger les contacts 12, 14, lorsque ledit stylo n'est pas utilisé.

Le capteur 6 selon l'invention a par exemple la forme d'une lamelle rectangulaire isolante que l'on peut introduire par l'une de ses extrémités référencée 19 dans la cavité 10 du stylo 4. On notera que ce capteur 6 est à usage unique.

Il comprend une électrode de mesure 20 et une électrode de référence 22 disposées, par exemple parallèlement longitudinalement sur le capteur 6. L'électrode de référence 22 comprend une bande 24 réalisée en matière électriquement conductrice. Cette bande 24 présente trois zones, une zone 26 dite de contact électrique, prévue vers l'extrémité 19 dudit capteur, une zone centrale 28 dite "piste conductrice" et une zone 30, prévue à l'autre extrémité du capteur et dite "collecteur de courant". De façon assez similaire, l'électrode de mesure 20 comprend une bande 32 réalisée en matière électriquement conductrice. Cette bande 32 comprend également trois zones, un contact électrique 34, une piste conductrice 36 et un collecteur de courant 37, recouvert contrairement au collecteur 30, d'un mélange 38.

Sur la figure 4, ce collecteur 37 n'est pas vraiment visible puisqu'il est caché par le mélange 38. On notera que dans chacune de ces électrodes, le collecteur et le conducteur de courant pourraient être en deux parties reliés électriquement entre elles et ne nécessitent pas obligatoirement d'être sous forme d'une bande unique 24 ou 32. Le mélange 38 comprend au moins une enzyme d'oxydo-réduction spécifique du composant à mesurer et au moins un médiateur transférant les électrons entre ladite enzyme et le collecteur de courant formé dans la bande 32.

De façon optionnelle, le mélange 38 précité peut également comprendre au moins une matière conductrice active et/ou au moins un additif qui sera décrit ultérieurement. Dans le cas où le mélange 38 comprend une matière conductrice active, le médiateur transfère les électrons entre l'enzyme et cette matière conductrice active qui à son tour, transfère les électrons vers le collecteur de courant.

La goutte 40 de l'échantillon de solution à tester, est déposée à cheval sur les deux électrodes 20 et 22 comme illustré en figure 4. Ainsi, le circuit électrique constitué par l'ampèremètre, les contacts 14 et 26, la piste conductrice 28, le collecteur 30, la goutte de solution 40, le mélange 38, le collecteur 37, la piste conductrice 36 et les contacts 34 et 12, est fermé.

L'appareil de mesure 2 qui vient d'être décrit est destiné à la réalisation de mesures in vitro, toutefois il est bien évident que le capteur 6 pourrait être utilisé in vivo, dans des appareils de mesure implantables. Dans ce cas, sa forme ou ses dimensions seraient adaptées à cette nouvelle application.

Par ailleurs, pour obtenir une précision accrue, on pourrait ajouter une deuxième électrode de mesure, identique à l'électrode de mesure 20 mais sans l'enzyme ou avec l'enzyme dénaturée.

La goutte 40 de solution à tester peut être de nature biologique, par exemple, du sang ou de l'urine d'un être humain ou d'un animal, ou un milieu de fermentation de micro-organismes. Elle peut éventuellement être d'origine synthétique, par exemple un tampon synthétique contenant des éléments à analyser.

On utilise comme enzyme d'oxydo-réduction, une enzyme spécifique du composant que l'on souhaite mesurer. De préférence selon l'invention, on utilise une enzyme choisie parmi les oxydases et les flavoprotéines. Lorsque l'on souhaite réaliser un capteur de glucose, on utilise la glucose oxydase GOD, par exemple une GOD présentant une activité d'environ 250 UI, obtenue à partir d'une moisissure d'Aspergillus niger.

La matière conductrice active utilisée de façon optionnelle, se présente de préférence sous forme d'une poudre de carbone, de graphite, d'or, de platine, de palladium ou d'oxyde de métal conducteur, par exemple d'oxyde de ruthénium ou sous forme d'un film de polymère conducteur, par exemple le polypyrrole. On utilisera de préférence une poudre de carbone.

Comme on l'a vu précédemment, on peut également ajouter un additif formant un réseau d'immobilisation de l'enzyme, du médiateur et/ou de la matière conductrice active sur la surface du collecteur 37 de l'électrode de mesure 20. Cet additif est par exemple, la sérumalbumine bovine (BSA), le glutaraldéhyde, le carbodiimide ou des polymères solubles dans l'eau.

Les bandes de matière électriquement conductrices 24, 32 sont réalisées par exemple, sous forme d'une couche d'un matériau choisi parmi l'or, l'argent, le platine, le palladium, le carbone, le graphite ou un oxyde de métal conducteur, tel qu'un oxyde de ruthénium, par exemple. De préférence, la bande 24 correspondant à l'électrode de référence 22 est réalisée en argent et la bande 32 correspondant à l'électrode de mesure 20 est réalisée en platine. Plus précisément, la partie de la bande 24 correspondant au collecteur de courant 30 est partiellement chlorurée.

On a découvert qu'une nouvelle famille de complexes d'osmium avec des ligands bipyridine substitué par au moins un groupe donneur d'électrons, présentait de bonnes propriétés de médiateur.

Le groupe donneur d'électrons est un groupe OH, un groupe alkoxy, un groupe aryloxy ou un groupe amine primaire, secondaire ou tertiaire.

Dans le cas d'un capteur de glucose et lorsqu'on utilise comme enzyme la glucose oxydase (GOD), on choisit de préférence parmi les médiateurs précités, le complexe tris (4,4' - diméthoxy - 2,2' - bipyridine) d'osmium ou le complexe bis (4,4' - diméthoxy - 2,2' - bipyridine) mono (4,4' - diméthyl - 2,2' - bipyridine) d'osmium.

Dans le cas d'un capteur de glucose, le mélange 38 dépose sur le collecteur de l'électrode de mesure 20 comprend 1 ml de tampon phosphate 10 mM ajusté à pH 6,8 : 1 à 1000 mg de poudre de carbone, de préférence 1 à 100 mg ou mieux 10 mg environ; 1 à 2000 UI de glucose oxydase par mg de poudre de carbone, de préférence 10 à 300 UI ou mieux 100 UI et 1 à 10000 µmol de médiateur par mg de poudre de carbone, de préférence 10 à 300 µmol ou mieux 50 µmol. Ce mélange est déposé à raison de 10 à 300 µl/cm$^2$ de surface active, de préférence 30 à 150 µl/cm$^2$ ou mieux 70 µl/cm$^2$.

Dans le capteur fini, séché, on peut donc considérer que le mélange 38 comprend 1 à 2000 UI de glucose oxydase par mg de poudre de carbone, de préférence 10 à 3000 UI ou mieux 100 UI et 1 à 10000 µmol de médiateur par mg de poudre de carbone, de préférence 10 à 300 µmol ou mieux 50 µmol.

Le capteur selon l'invention, muni des médiateurs précités, présente un certain nombre de propriétés variant en fonction des ligands utilisés et des substitutions réalisées sur ces ligands.

Plusieurs expériences ont été réalisées qui prouvent les performances et l'efficacité de ces nouveaux médiateurs et qui donnent les conditions d'optimalisation des divers éléments constituant l'électrode de mesure. Ces expériences sont décrites ci-après.

Expérience 1 :

Mesures de différents médiateurs par voltamétrie cyclique.

a) mesures effectuées avec le complexe tris (4,4' - diméthoxy - 2,2' - bipyridine) d'osmium.

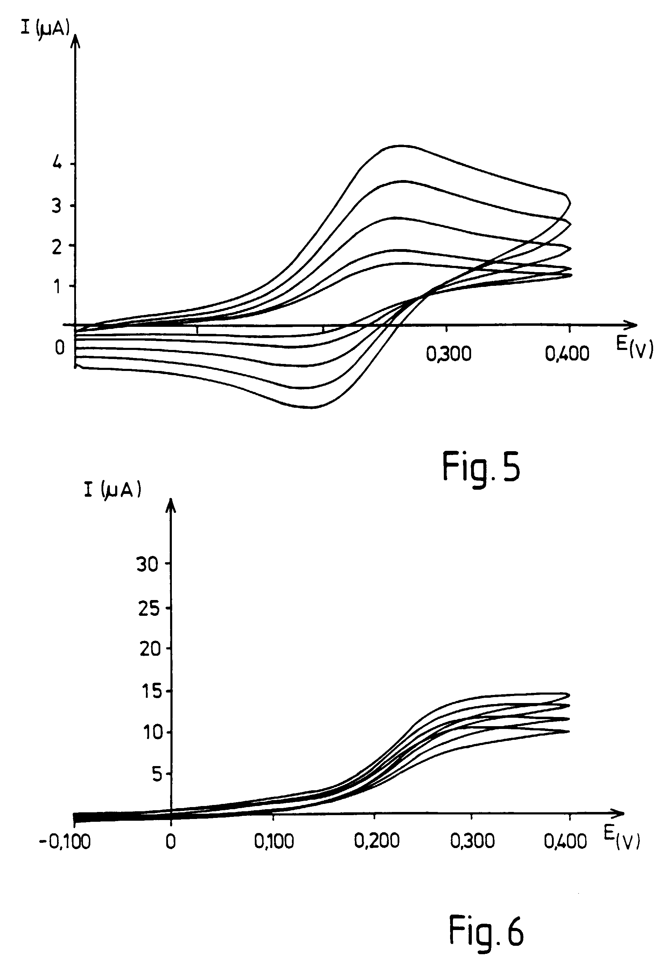

Le complexe précité a été testé par voltamétrie cyclique en courant continu, afin de déterminer d'une part son potentiel normal d'oxydo-réduction E° et d'autre part la constante de vitesse k. Cette constante k correspond à la réaction de transfert d'électrons, à partir de la GOD, vers le médiateur. La voltamétrie cyclique consiste à disposer dans la solution à analyser, une électrode de travail, une contre-électrode et une électrode de référence, puis à effectuer un balayage, à vitesse constante et entre deux bornes, du potentiel de l'électrode de travail et à mesurer l'intensité du courant obtenu. Les courbes des figures 5 et 6 représentent les résultats obtenus par cette méthode. Ces expériences ont été réalisées avec une électrode de travail en carbone vitreux, une électrode de référence au calomel, une contre-électrode en platine et une cellule électrochimique de 5 à 20 ml. Les mesures ont été réalisées dans un tampon phosphate PBS (NaCl 100mM, NaH$_2$PO$_4$ 10mM, ajusté à pH 7,4; EDTA (acide éthylène tétraacétique) 0,1mM; PMSF (fluorure de phénylméthylsulfonate) 0,01mM et avec une concentration du complexe précité de 5.10$^{-4}$M. On a utilisé différentes vitesses de balayage du potentiel :5, 10, 25, 50 et 100 mV.s$^{-1}$. On obtient les courbes de la figure 5 et une valeur de E° de 225 mV. L'addition d'une solution saturée de glucose n'a aucun effet sur les courbes de la figure 5, ce qui est normal puisqu'il n'y a pas la glucose oxydase (GOD).

En revanche, l'addition de GOD (en quantité supérieure à 5.10$^{-8}$ M de préférence 4.10$^{-6}$ M) permet d'obtenir les courbes de la figure 6, présentant une forme dite "vague catalytique" caractéristique. Dans cette figure 6, on a utilisé comme vitesse de balayage du potentiel : 10, 25, 50 et 100 mV.s$^{-1}$.

On obtient une première réaction :

$$\text{Médiateur}_{(oxydé)} + \text{GOD}_{(réduite)} \xrightarrow{k} \text{Médiateur}_{(réduit)} + \text{GOD}_{(oxydée)}$$

qui est irréversible, (avec une constante k), et une seconde réaction :

$$\text{Médiateur}_{(réduit)} + e^{-} \rightarrow \text{Médiateur}_{(oxydé)}$$

qui est électrochimiquement réversible et extrêmement rapide.

Le médiateur réalise un transfert électrochimiquement réversible d'un électron vers les collecteurs de courant précédemment décrits.

Au cours de la première réaction, on peut mesurer la constante de second ordre k. Pour le complexe étudié ici, on trouve $k = 2,5.10^{6} \pm 0,5 \ M^{-1}.s^{-1}$.

b) mesures effectuées avec d'autres complexes.

Des expériences similaires à celles qui viennent d'être décrites ont été réalisées pour d'autres complexes. Le tableau 1 donne les valeurs des constantes de vitesse k trouvées et des potentiels normaux d'oxydo-réduction E° en mV par rapport à une électrode de référence au calomel (SCE).

## TABLEAU 1

| | Complexes | $E°(mV_{/SCE})$ | $k(M^{-1}.s^{-1})$ |
|---|---|---|---|
| 1 | complexe tris (4,4' - diméthoxy - 2,2' - bipyridine) d'osmium | 225 | $2,5.10^{6}$ |
| 2 | complexe bis (4,4' - diméthoxy - 2,2' - bipyridine) mono (4,4' - diméthyl - 2,2' - bipyridine) d'osmium | 340 | $2.10^{6}$ |

| | | | |
|---|---|---|---|
| 3 | complexe bis (4,4' - diméthyl - 2,2' - bipyridine) mono (4,4' - diméthoxy - 2,2' - bipyridine) d'osmium | 390 | N.D |
| 4 | complexe mono (4,4' - diméthoxy - 2,2' - bipyridine) mono (4,4' - dihydroxy - 2,2' - bipyridine) mono (4,4' - diméthyl - 2,2' - bipyridine) d'osmium | | |
| | pH<4,5 | 340 | N.D |
| | pH>4,5 | 190 | $2.10^5$ |
| 5 | complexe tris (4,4' - diméthyl - 2,2' - bipyridine) d'osmium | 425 | $1,5.10^6$ |
| 6 | complexe tris (4,4' - dihydroxy - 2,2' - bipyridine) d'osmium | -1000 | $\sim 0$ |
| 7 | complexe tris (4,4'- diamino - 2,2' - bipyridine) de ruthénium | 170 | $1,6.10^6$ |
| 8 | complexe tris (4,4' - diamino - 2,2' - bipyridine) de fer | 70 | $1,4.10^5$ |

N.D. signifie non-déterminé.

A la lecture de ce tableau 1, on constate que la famille de médiateurs présente une très large gamme de potentiels rédox, variant entre - 1000 mV et + 425 mV (par rapport à une électrode de référence au calomel SCE). La limite inférieure de cette gamme est beaucoup plus basse que tous les potentiels rédox des médiateurs décrits jusqu'à présent dans la littérature. Par ailleurs, cette gamme de potentiels est également beaucoup plus large que celle que l'on obtenait avec la famille des ferrocènes. Ceci est dû au nombre important de substituants que l'on peut utiliser et au plus grand nombre de combinaisons de substitutions possibles.

La constante de second ordre $k_f$ correspondant à la constante de vitesse de la réaction d'oxydo-réduction entre l'enzyme et le médiateur selon l'invention, est beaucoup plus rapide qu'avec les autres médiateurs connus jusqu'à présent et est plus rapide qu'avec l'oxygène. En effet, l'oxygène présente une constante k de $1,5.10^6 M^{-1}.s^{-1}$ seulement. Ceci limite les problèmes précédemment décrits de compétition de l'oxygène avec le médiateur lors de la réaction de transfert d'électrons à partir de la GOD. -De même, les autres réactions de compétition se réalisant beaucoup plus lentement n'influencent pas le résultat de l'appareil de mesure.

En conséquence, on a sélectionné comme médiateurs pour les capteurs de glucose pour illustrer la suite de la description, les complexes 1 et 2 qui présentent les caractéristiques les plus favorables, à savoir simultanément une constante k élevée et un faible potentiel normal d'oxydo-réduction E° néanmoins supérieur à -300 mV, ce qui correspond au potentiel normal du groupe $FAD/FADH_2$ de la GOD.

<u>Expérience 2</u> :

<u>Optimalisation des différents composants du mélange de l'électrode de mesure.</u>

On a essayé de déterminer les quantités optimales respectives des différents constituants du mélange déposé sur le collecteur de l'électrode de mesure.

Ceci a été réalisé en fabriquant un mélange 38 comprenant l'un des deux complexes préférés précédemment cités, de la poudre de carbone, la glucose oxydase immobilisée et comme additif la sérumalbumine bovine et le glutaraldéhyde puis en déposant sur la partie collecteur de courant 37 de la bande électriquement conductrice 36 une quantité de 70 $\mu$l de ce mélange par cm$^2$ afin de constituer une électrode de mesure 20. On a ensuite réalisé différents types d'électrodes de mesure en faisant varier graduellement l'un des composants du mélange et en maintenant les autres constants.

Les différents capteurs préparés de cette façon ont été utilisés pour des mesures potentiostatiques, à un potentiel de 300 mV, dans de multiples échantillons de sang contenant diverses quantités de glucose. Les résultats sont illustrés ci-après.

a) <u>optimalisation de la quantité de poudre de carbone.</u>

On a réalisé plusieurs types de capteurs différents (mais on a choisi d'en présenter seulement trois), en mélangeant dans 3 ml de tampon phosphate PBS, une quantité constante de GOD, (36,9 mg), une quantité constante de complexe bis (4,4' - diméthoxy - 2,2'-bipyridine) mono (4,4' - diméthyl - 2,2' - bipyridine) d'osmium (3,0 mg), utilisé comme médiateur, une quantité constante de glutaraldéhyde à 25 % (25 $\mu$l), une quantité constante de sérumalbumine bovine à 15 % (290 $\mu$l) et respectivement 25, 50 ou 250 mg de poudre de carbone.

Le tampon phosphate PBS utilisé ici et dans les expériences ci-dessous mentionnées est un tampon 10 mM ajusté à pH 6,8.

Ensuite on a testé ces trois types de capteur dans une solution physiologique contenant des quantités différentes de glucose (entre 0 et 20 mM de glucose) et on a mesuré la densité de courant obtenue au bout de 30 secondes ($D_{30}$). La solution physiologique est composée de NaCl 115 mM, de KCl 25 mM, de $K_2HPO_4$. $3H_2O$ 5 mM et de $KH_2PO_4$ 0,5 mM.

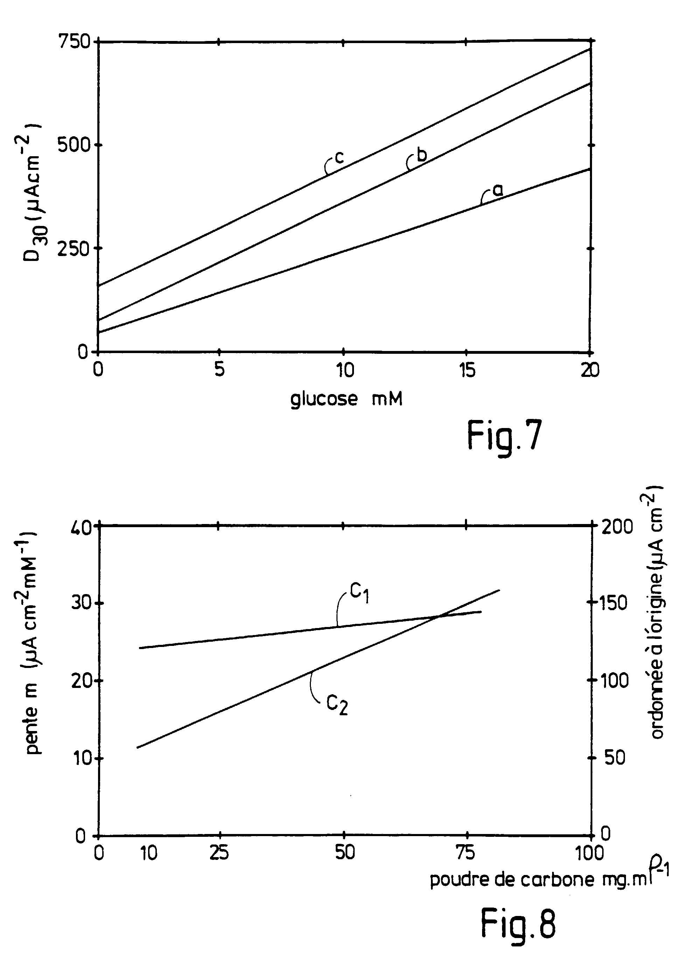

Les résultats obtenus sont illustrés en figure 7 où les droites a, b, c correspondent respectivement aux résultats observés avec des capteurs contenant 25, 50 et 250 mg de carbone dans 3 ml de tampon phosphate PBS, soit des concentrations approximatives de 8, 17 et 83 mg par ml. La encore on notera que l'on a effectué beaucoup plus de mesures, mais que l'on a choisi de représenter uniquement les droites a, b, c.

On a ensuite calculé la pente (m) de toutes les droites représentant la totalité des mesures effectuées et on a reporté ces valeurs en figure 8 (courbe $C_1$) où l'axe des abscisses représente la quantité de carbone en mg par ml de tampon phosphate PBS. De même, on a calculé l'ordonnée à l'origine de ces droites et on a reporté ces valeurs en figure 8 (courbe $C_2$). L'ordonnée à l'origine correspond à la valeur du point d'intersection d'une droite de la figure 7 et de l'axe des ordonnées, c'est-à-dire à la valeur du courant résiduel.

On constate que la courbe $C_1$ est sensiblement horizontale entre 17 et 83 mg de carbone, ce qui signifie qu'entre ces deux valeurs, la quantité de carbone influence peu les résultats du capteur. Toutefois, comme une mince couche de carbone présente de meilleures propriétés mécaniques et diffusionnelles, on préfère utiliser le moins possible de carbone. De plus, on constate que la valeur de l'ordonnée à l'origine de la droite a (8 mg de carbone par ml) est la plus faible, ce qui signifie que l'on a le plus faible courant résiduel.

En conséquence, on préfère utiliser environ 10 mg de carbone par ml de tampon phosphate PBS.

b) <u>optimalisation de la quantité d'enzyme (GOD).</u>

On a réalisé plusieurs types de capteurs différents, (mais on a choisi de n'en représenter que trois), en mélangeant dans 3 ml de tampon phosphate PBS, une quantité constante de carbone (25 mg), une quantité constante (3 mg) du même médiateur que celui du paragraphe a) des quantités constantes de glutaraldéhyde à 25 % (25 $\mu$l) et de sérumalbumine bovine à 15 % (290 $\mu$l) et respectivement des quantités de 2175, 4375 et 8750 UI de glucose oxydase GOD, soit des concentrations respectives en GOD de 87, 175 et 350 UI de glucose oxydase (GOD) par mg de carbone.

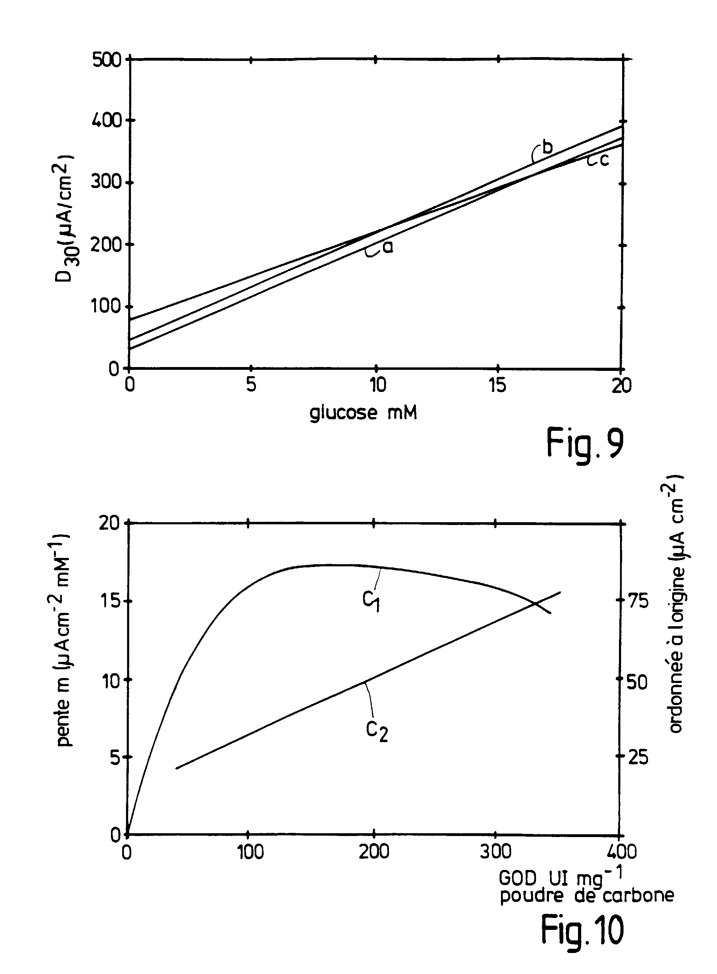

Ensuite on a effectué les mêmes séries de mesures et de calculs qu'au paragraphe a). Les droites a, b, c de la figure 9 correspondent respectivement aux résultats observés avec des capteurs contenant 87, 175 et 350 UI de glucose oxydase par mg de poudre de carbone. Les courbes $C_1$ et $C_2$ de la figure 10 représentent respectivement la pente (m) et l'ordonnée à l'origine. L'axe des abcisses de la figure 10 exprime la quantité de GOD en UI par mg de poudre de carbone.

On remarque qu'entre 75 et 350 UI de GOD par mg de poudre de carbone, la courbe $C_1$ est sensiblement hori-

zontale, ce qui signifie qu'entre ces deux valeurs la quantité de GOD influence peu les résultats. Par ailleurs, l'ordonnée à l'origine de la droite a est la plus faible ce qui signifie qu'on a le plus faible courant résiduel.

En conséquence, on préfère utiliser environ 100 UI de GOD par mg de poudre de carbone.

c) optimalisation de la quantité de médiateur.

On a réalisé trois types de capteurs différents en mélangeant dans 3 ml de tampon phosphate PBS, une quantité constante de carbone (25 mg), une quantité constante de GOD (36,9 mg), des quantités constantes de glutaraldéhyde à 25 % (25 µl) et de sérumalbumine bovine à 15 % (290 µl) et respectivement 825; 1675 et 3325 µmol de complexe bis (4,4' - diméthoxy - 2,2' - bipyridine) mono (4,4'-diméthyl - 2,2' - bipyridine) d'osmium, soit des concentrations en médiateur de 33; 67 et 133 µmol par mg de poudre de carbone.

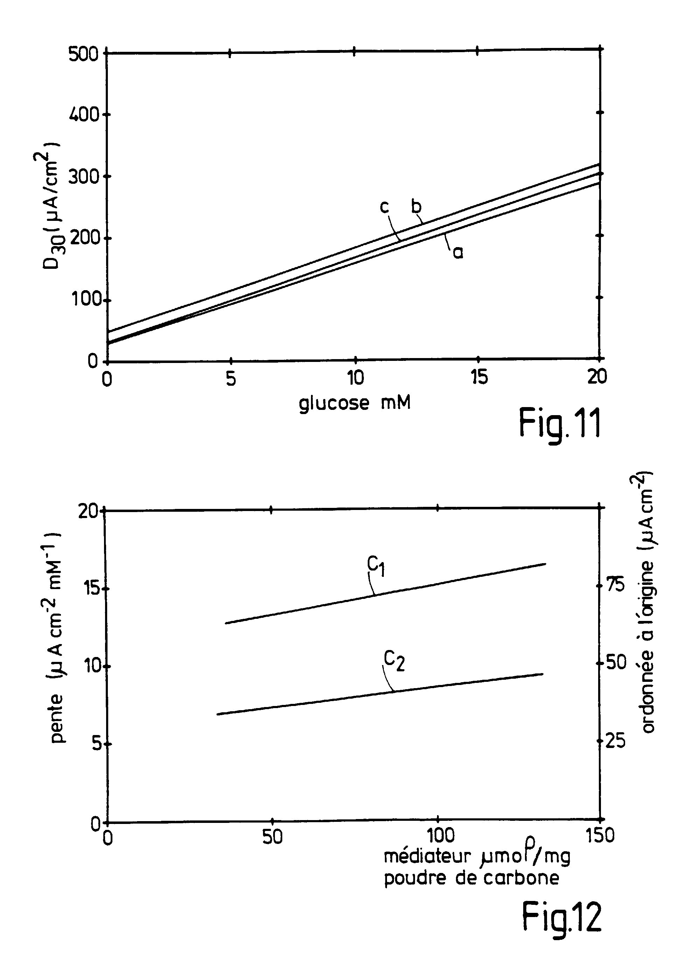

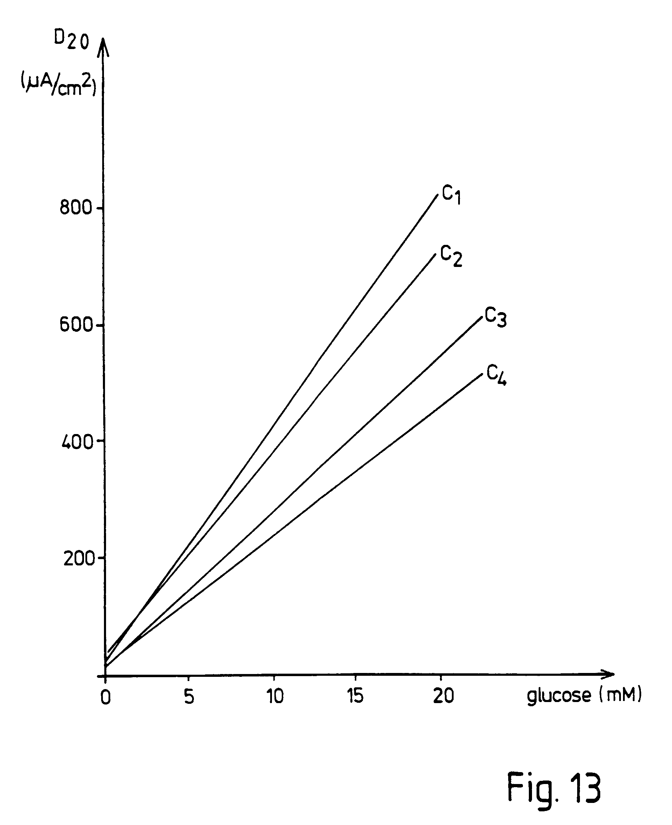

Ensuite, on a effectué les mêmes séries de mesures et de calculs qu'au paragraphe a). Les droites a, b, c de la figure 11 correspondent respectivement aux résultats observés avec 33; 67 et 133 µmol de ce complexe par mg de carbone. Les courbes $C_1$ et $C_2$ de la figure 12 représentent respectivement la pente (m) et l'ordonnée à l'origine. L'axe des abscisses de la figure 12 représente la quantité de médiateur en µmol par mg de poudre de carbone.

On remarque que les courbes $C_1$ et $C_2$ sont sensiblement horizontales. Pour des valeurs de médiateur inférieures à 50 µmol, on est obligé d'effectuer les mesures à un potentiel supérieur à 300 mV. Comme on préfère travailler à un potentiel le plus faible possible, on préfère donc utiliser environ 50 µmol de médiateur par mg de poudre de carbone.

Les optimalisations qui ont été réalisées pour le complexe bis (4,4' - diméthoxy - 2,2' - bipyridine) mono (4,4' - diméthyl - 2,2' - bipyridine) d'osmium sont également valables pour le complexe tris (4,4 - diméthoxy - 2,2' - bipyridine) d'osmium.

Expérience 3 :

Etalonnage du capteur dans du sang et du tampon.

Les courbes de la figure 13 illustrent des mesures potentiostatiques effectuées avec des capteurs présentant comme médiateur les deux complexes préférés de l'invention et en faisant varier la concentration de glucose dans des échantillons de sang ou de tampon phosphate PBS. Les mesures ont été effectuées à 300 mV et la lecture de la densité de courant D20 a été faite après 20 secondes.

Les courbes $C_1$ et $C_3$ correspondent respectivement à des mesures effectuées dans le tampon phosphate et dans le sang avec un capteur utilisant le complexe tris (4,4' - diméthoxy - 2,2'- bipyridine) d'osmium, tandis que les courbes $C_2$ et $C_4$ correspondent respectivement à des mesures effectuées dans le tampon phosphate et dans le sang avec un capteur utilisant le complexe bis (4,4' - diméthoxy - 2,2' - bipyridine) mono (4,4' - diméthyl - 2,2' - bipyridine) d'osmium.

Comme on peut le constater sur la figure 13, les différentes courbes présentent un tracé linéaire et une pente suffisamment importante jusqu'à des valeurs de 20 mM de glucose. En conséquence, chez un patient où les valeurs physiologiques du glucose peuvent varier typiquement de 3 à 20 mM, on peut utiliser de façon fiable le capteur selon l'invention, puisqu'à une faible variation de la concentration en glucose correspond une variation suffisante de la densité de courant mesurée.

Les différences observées entre les mesures effectuées dans du tampon PBS et du sang complet sont dues au même phénomène que celui décrit notamment dans (Fogh-Andersen, N. et ses collaborateurs (1990), Clin. Chim. Acta 189, 33-38), pour le plasma et le sang complet. Cette différence est principalement due au volume occupé par les protéines dans le sang complet.

Expérience 4 :

Influence de l'hématocrite sur les résultats fournis par le capteur.

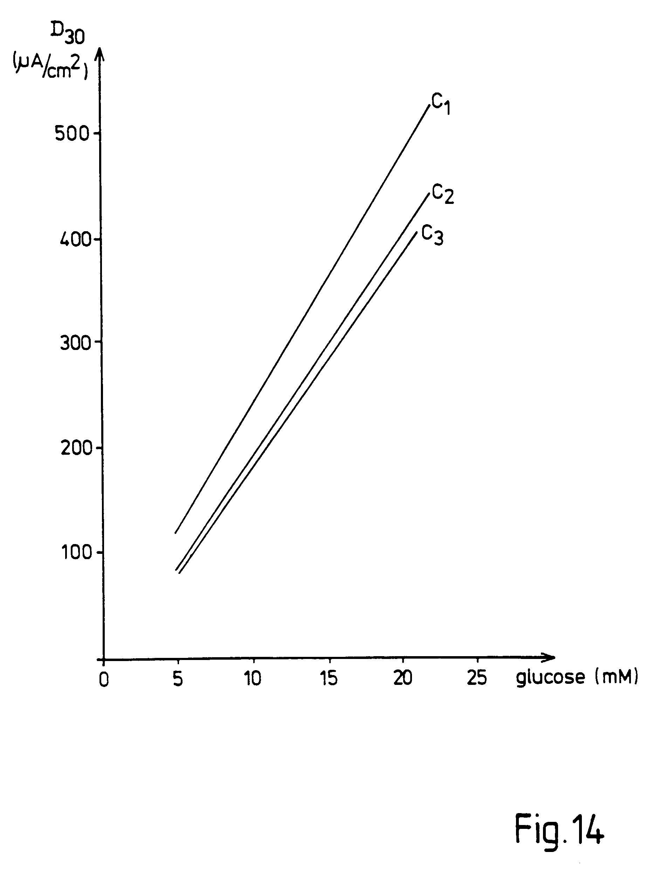

Les courbes de la figure 14 illustrent les variations de la densité de courant ($D_{30}$) obtenue après 30 secondes, en fonction de la concentration en glucose dans du sang humain artificiellement reconstitué. Les échantillons de sang ont été préparés de la façon suivante. On a séparé le plasma et les cellules sanguines par centrifugation à 3000 tours par minute, pendant 15 minutes à 4°C. Ensuite, on a reconstitué le sang de façon à obtenir diverses valeurs d'hématocrite (0,35; 0,50 et 0,60) et on a ajouté à ces échantillons certaines quantités de glucose. La concentration en glucose a été mesurée en utilisant un appareil de laboratoire calibré, par exemple l'appareil référencé 23A, (disponible chez Yellow Springs Instrument, Yellow Springs, OHIO). Les mesures potentiostatiques ont été effectuées à 300 mV avec des capteurs présentant comme médiateur le complexe bis (4,4' - diméthoxy - 2,2' - byipridine) mono (4,4' - diméthyl - 2,2' - bipyridine d'osmium. Les mesures de la densité de courant ont été effectuées après 30 secondes.

Les courbes $C_1$, $C_2$ et $C_3$ correspondent respectivement à des échantillons contenant 35 % de cellules et 65 % de plasma, 50 % de cellules et 50 % de plasma et 60 % de cellules et 40 % de plasma.

La courbe $C_2$ correspond à un hématocrite normal. On constate que la courbe $C_3$ (hématocrite 0,60) correspondant à un hématocrite élevé, ne diffère pratiquement pas de la courbe $C_2$.

En revanche, la courbe $C_1$ (hématocrite 0,35) correspondant à l'hématocrite d'une personne anémiée diffère de la courbe $C_2$.

En conséquence, le capteur selon l'invention donne des résultats fiables chez un patient présentant un hématocrite élevé mais moins fiables chez une personne présentant une anémie.

Expérience 5 :

Influence du pH sur l'activité de médiateur du complexe tris (4,4' -diméthoxy - 2,2' - bipyridine) d'osmium et du complexe bis (4,4'-diméthoxy - 2,2' - bipyridine) mono (4,4' - diméthyl - 2,2'-bipyridine) d'osmium

Ces deux complexes ont été mélangés dans une solution de tampon phosphate PBS dont on a fait varier le pH et dont on a mesuré le potentiel normal d'oxydo-réduction E°.

On observe un potentiel E° stable pour un pH compris entre 1 et 12. Ce potentiel E° est de + 225 mV pour le premier complexe et de + 340 mV pour le second. Comme dans la pratique, le pH du sang humain est d'environ 7,4, de faibles variations du pH sanguin n'affectent pas le résultat de glycémie donné par le capteur selon l'invention.

Expérience 6 :

Influence de la présence de certains médicaments, sur les résultats fournis par le capteur.

Enfin, une dernière série d'expériences a été effectuée afin de vérifier si les résultats fournis par ce capteur pouvaient être influencés par la présence de médicaments présents dans le sang au moment de la mesure. En effet, un patient peut parfaitement avoir ingéré des médicaments comme l'aspirine ou la vitamine C avant d'effectuer une mesure de glycémie.

En conséquence, on a testé l'influence possible de l'acide acétylsalicylique, de l'acétaminophénol et de l'acide ascorbique sur les résultats fournis par le capteur selon l'invention.

Les expériences ont été réalisées avec un capteur utilisant comme médiateur le complexe tris (4,4' - diméthoxy - 2,2' - bipyridine) d'osmium.

Les mesures potentiostatiques ont été effectuées à 300 mV. La lecture de la densité de courant ($D_{30}$) a été effectuée après 30 secondes. Les différentes courbes représentent les variations de la densité de courant en fonction de la concentration en glucose, lorsque différentes quantités de chacun des médicaments testés sont présents dans un échantillon de solution physiologique.

Les résultats obtenus sont les suivants :

-    acétaminophenol :

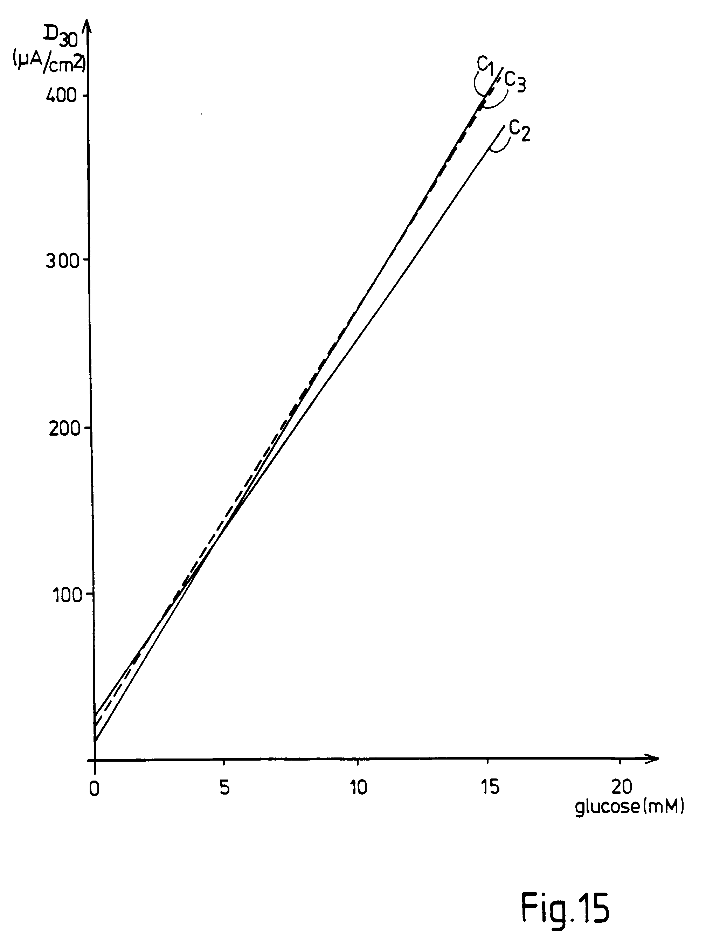

La figure 15 illustre les courbes obtenues. Les courbes $C_1$, $C_2$ (en pointillés) et $C_3$ correspondent respectivement à des concentrations de 0, 50 et 500 $\mu$M d'acétaminophénol.

La valeur de 50 $\mu$M correspond à ce que l'on retrouve chez un patient ayant absorbé de l'acétaminophénol selon une posologie normale, en revanche la valeur de 500 $\mu$M correspond à un excès. On peut constater qu'entre 4 et 10 mM de glucose (ce qui correspond sensiblement aux valeurs physiologiques), la présence de ce médicament n'a guère d'influence sur les résultats fournis par ce capteur, puisque toutes les courbes sont sensiblement superposées.

-    acide ascorbique :

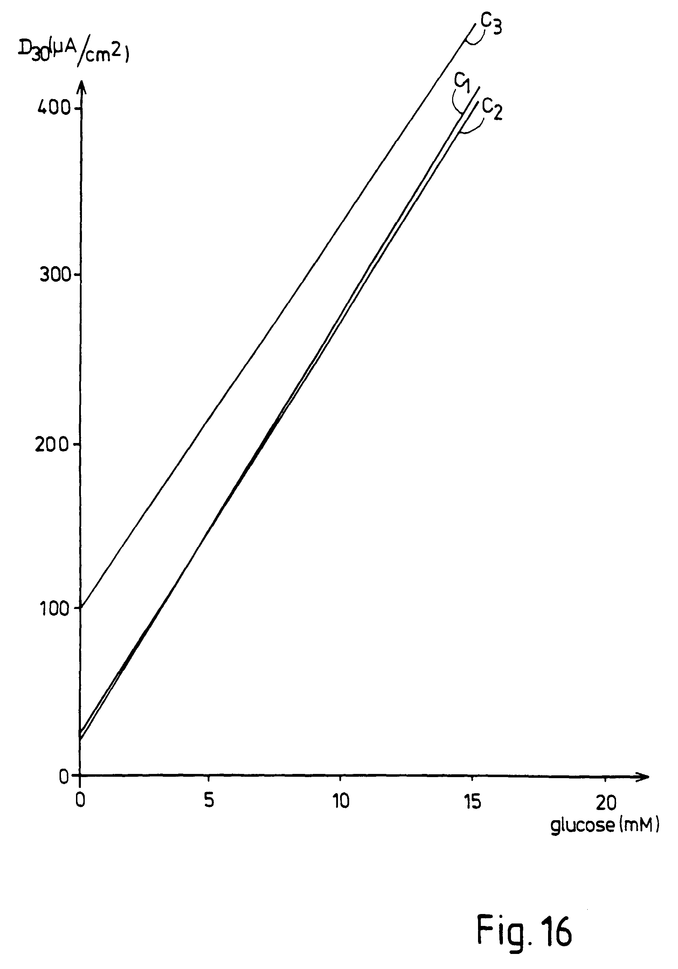

La figure 16 illustre les courbes obtenues. Les courbes $C_1$, $C_2$ et $C_3$ correspondent respectivement à des concentrations de 0, 100 et 1000 $\mu$M d'acide ascorbique par ml de sang.

La valeur de 100 $\mu$M (courbe $C_2$) correspond aux valeurs que l'on trouve chez un patient ayant absorbé de la vitamine C selon une posologie normale, par contre la valeur de 1000 $\mu$M (courbe $C_3$) correspond à un excès d'acide ascorbique.

On constate donc que lors de la présence d'acide ascorbique en excès (courbe $C_3$), toutes les valeurs de concentration en glucose sont augmentées par rapport à la normale. Au contraire, la courbe $C_2$ est sensiblement identique à $C_1$ et à des valeurs physiologiques, on remarque que la présence d'acide ascorbique n'affecte pas les résultats fournis par le capteur.

-    acide acétylsalicylique

Il n'a pas été jugé nécessaire de présenter une figure illustrant les résultats obtenus, puisqu'on a constaté qu'une quantité d'acide acétylsalicylique allant jusqu'à 25 mM donnait des droites sensiblement identiques à celle correspondant à une quantité de 0 mM d'acide acétylsalicylique. En conséquence, on en déduit que la présence d'acide acétylsalicylique n'affecte pas les résultats fournis par le capteur.

**Revendications**

1. Capteur de mesure de la quantité d'un composant en solution, comprenant :

    - au moins une électrode de mesure (20) et une électrode de référence (22), isolées l'une de l'autre et destinées à venir en contact avec ladite solution, lesdites électrodes (20, 22) comprenant respectivement des contacts électriques (34; 26) adaptés pour être branchés sur un dispositif d'exploitation (4) du signal fourni par ledit capteur,
    - l'électrode de mesure (20) comprenant au moins un collecteur de courant relié (37) électriquement à l'un des contacts électriques (34) et recouvert d'un mélange (38) comprenant au moins une enzyme d'oxydo-réduction spécifique dudit composant et au moins un médiateur transférant les électrons entre ladite enzyme et ledit collecteur de courant, caractérisé en ce que le médiateur est choisi parmi les complexes osmium avec des ligands bipyridine substitués par au moins un groupe donneur d'électrons choisi parmi le groupe OH, un groupe alkoxy, un groupe aryloxy ou un groupe amine primaire, secondaire ou tertiaire.

2. Capteur selon la revendication 1, caractérisé en ce que le médiateur est choisi parmi le complexe tris (4,4' - diméthoxy - 2,2' - bipyridine) d'osmium ou le complexe bis (4,4' - diméthoxy - 2,2' - bipyridine) mono (4,4' diméthyl - 2,2' - bipyridine) d'osmium.

3. Capteur selon la revendication 1, caractérisé en ce que le mélange (38) de l'électrode de mesure (20) comprend en outre une matière conductrice active et en ce que le médiateur transfère les électrons entre l'enzyme et cette matière conductrice active.

4. Capteur selon la revendication 1, caractérisé en ce que l'enzyme d'oxydo-réduction est choisie parmi les oxydases ou les flavoprotéines.

5. Capteur selon la revendication 4 pour mesurer la quantité de glucose présente dans la solution, caractérisé en ce que l'enzyme est la glucose-oxydase.

6. Capteur selon la revendication 3, caractérisé en ce que la matière conductrice active est une poudre d'un matériau choisi parmi le carbone, l'or, le platine, le palladium ou un oxyde de métal conducteur.

7. Capteur selon la revendication 3, caractérisé en ce que la matière conductrice est un film d'un polymère conducteur.

8. Capteur selon la revendication 3, caractérisé en ce que le mélange (38) de l'électrode de mesure (20) comprend un additif formant un réseau d'immobilisation de l'enzyme, du médiateur et/ou de la matière conductrice active sur la surface du collecteur (37) de l'électrode de mesure (20).

9. Capteur selon la revendication 8, caractérisé en ce que l'additif est choisi parmi la sérumalbumine bovine, le glutaraldéhyde, le carbodiimide ou des polymères solubles dans l'eau.

10. Capteur selon les revendications 2, 5 et 6, caractérisé en ce que le mélange (38) déposé sur le collecteur de l'électrode de mesure (20) comprend entre 1 et 2000 UI de glucose oxydase par mg de poudre de carbone et entre 1 et 10000 µmol de médiateur par mg de poudre de carbone.

11. Capteur selon la revendication 10, caractérisé en ce que le mélange (38) déposé sur le collecteur de l'électrode de mesure (20) comprend entre 10 et 300 UI de glucose oxydase par mg de poudre de carbone et entre 10 et 300 µmol de médiateur par mg de poudre de carbone.

12. Capteur selon la revendication 11, caractérisé en ce que le mélange (38) déposé sur le collecteur de l'électrode de mesure (20) comprend environ 100 UI de glucose oxydase par mg de poudre de carbone et environ 50 µmol de médiateur par mg de poudre de carbone.

13. Ensemble caractérisé en ce qu'il est composé d'un capteur selon l'une quelconque des revendications 1 à 12 et d'un dispositif d'exploitation du signal fourni par ledit capteur, ce dispositif comprenant au moins deux contacts électriques conçus pour être reliés à au moins deux électrodes dudit capteur, un ampèremètre et des moyens d'affichage des résultats.

**Patentansprüche**

1. Meßfühler für die Quantität einer in Lösung befindlichen Komponente, umfassend:

   - mindestens eine Meßelektrode (20) und eine Referenzelektrode (22), die voneinander isoliert sind und dazu bestimmt sind, mit der Lösung in Kontakt zu gelangen, welche Elektroden (20, 22) jeweils elektrische Kontakte (34; 26) umfassen, ausgebildet, um an eine Auswerteeinrichtung (4) des von dem Fühler gelieferten Signals angeschlossen zu werden,
   - welche Meßelektrode (20) mindestens einen Stromkollektor umfaßt, elektrisch verbunden (37) mit einem der elektrischen Kontakte (34) und beschichtet mit einem Gemisch (38), das mindestens ein Oxydoreduktionsenzym, das für die Komponente spezifisch ist, umfaßt, sowie mindestens einen Mittler, der die Elektronen zwischen dem Enzym und dem Stromkollektor transferiert, dadurch gekennzeichnet, daß der Mittler unter den Osmiumkomplexen mit Bipyridinliganden, substituiert durch mindestens eine elektronenliefernde Gruppe, gewählt unter der Gruppe OH, einer Alkoxygruppe, einer Aryloxygruppe oder einer Gruppe primärer, sekundärer oder tertiärer Amine, gewählt ist.

2. Fühler nach Anspruch 1, dadurch gekennzeichnet, daß der Mittler aus dem Osmiumkomplex tris- (4,4' - Dimethoxy - 2,2' - Bipyridin) oder dem Osmiumkomplex bis- (4,4' - Dimethoxy - 2,2' - Bipyridin)-mono-(4,4' Dimethyl - 2,2' - Bipyridin) gewählt ist.

3. Fühler nach Anspruch 1, dadurch gekennzeichnet, daß das Gemisch (38) der Meßelektrode (20) ferner ein aktiv leitendes Material umfaßt und daß der Mittler die Elektronen zwischen dem Enzym und diesem aktiv leitenden Material transferiert.

4. Fühler nach Anspruch 1, dadurch gekennzeichnet, daß das Oxydoreduktionsenzym unter den Oxydasen oder Flavoproteinen gewählt ist.

5. Fühler nach Anspruch 4 zum Messen der Glucosequantität, die in der Lösung vorhanden ist, dadurch gekennzeichnet, daß das Enzym die Glucoseoxydase ist.

6. Fühler nach Anspruch 3, dadurch gekennzeichnet, daß das aktive leitende Material ein Pulver eines Materials ist, gewählt unter Kohlenstoff, Gold, Platin, Palladium oder einem leitenden Metalloxid.

7. Fühler nach Anspruch 3, dadurch gekennzeichnet, daß das leitende Material eine Folie eines leitenden Polymers ist.

8. Fühler nach Anspruch 3, dadurch gekennzeichnet, daß das Gemisch (38) der Meßelektrode (20) ein Additiv umfaßt, das ein Immobilisierungsnetz des Enzyms, des Mittlers und/oder des aktiven leitenden Materials auf der Oberfläche des Kollektors (37) der Meßelektrode (20) bildet.

9. Fühler nach Anspruch 8, dadurch gekennzeichnet, daß das Additiv aus dem Rinderserumalbumin, dem Glutaraldehydrid, dem Carbodiimid oder den wasserlöslichen Polymeren gewählt ist.

10. Fühler nach Ansprüchen 2, 5 und 6, dadurch gekennzeichnet, daß das auf dem Kollektor der Meßelektrode (20) aufgebrachte Gemisch (38) zwischen 1 und 2000 IE an Glucoseoxydase pro mg Kohlenstoffpulver und zwischen 1 und 1000 µmol Mittler pro mg Kohlenstoffpulver umfaßt.

11. Fühler nach Anspruch 10, dadurch gekennzeichnet, daß das auf den Kollektor der Meßelektrode (20) aufgebrachte Gemisch (38) zwischen 10 und 300 IE an Glucoseoxydase pro mg Kohlenstoffpulver und zwischen 10 und 300 µmol Mittler pro mg Kohlenstoffpulver umfaßt.

12. Fühler nach Anspruch 11, dadurch gekennzeichnet, daß das auf den Kollektor der Meßelektrode (20) aufgebrachte Gemisch (38) etwa 100 IE Glucoseoxydase pro mg Kohlenstoffpulver und etwa 50 μmol Mittler pro mg Kohlenstoffpulver umfaßt.

13. Baugruppe, dadurch gekennzeichnet, daß sie aus einem Fühler gemäß einem der Ansprüche 1 bis 12 und einer Auswerteeinrichtung für das von dem Fühler gelieferte Signal besteht, welche Einrichtung mindestens zwei elektrische Kontakte, die mit mindestens zwei Elektroden des Fühlers in Verbindung bringbar sind, ein Amperemeter und Mittel zur Anzeige des Ergebnisses umfaßt.

**Claims**

1. Sensor for measuring the amount of a component in a solution comprising :

   - at least one measuring electrode (20) and one reference electrode (22) insulated from one another and adapted to come into contact with said solution, said electrodes (20, 22) comprising respectively electrical contacts (34; 26) adapted to be connected to a device (4) for processing the signal supplied by said sensor,
   - the measuring electrode (20) comprising at least one current collector (37) electrically connected to one of the electrical contacts (34) and coated with a mixture (38) comprising at least one oxidation-reduction enzyme specific to said component and at least one mediator transferring the electrons between said enzyme and said current collector, characterized in that the mediator is chosen from among the complexes of osmium with bipyridine ligands substituted by at least one electron donor group selected hydroxy, alkoxy, aryloxy or primary, secondary or tertiary amine groups.

2. Sensor according to claim 1, characterized in that the mediator is tris(4,4'-dimethoxy-2,2'-bipyridine) osmium or bis(4,4'-dimethoxy-2,2'-bipyridine)mono(4,4'-dimethyl-2,2'-bipyridine) osmium.

3. Sensor according to claim 1, characterized in that the mixture (38) of the measuring electrode (20) also comprises an active conducting material and in that the mediator transfers the electrons between the enzyme and this active conducting material.

4. Sensor according to claim 1, characterized in that the oxidation-reduction enzyme is an oxidase or a flavoprotein.

5. Sensor according to claim 4 for the measurement of the amount of glucose present in the solution, characterized in that the enzyme is glucose oxidase.

6. Sensor according to claim 3, characterized in that the active conducting material is a powder of carbon, gold, platinum, palladium or a conducting metal oxide.

7. Sensor according to claim 3, characterized in that the active conducting material is a film of a conducting polymer.

8. Sensor according to claim 3, characterized in that the mixture (38) of the measuring electrode (20) comprises an additive forming an immobilization network of the enzyme, of the mediator and/or of the active conducting material on the surface of the collector (37) of the measuring electrode (20).

9. Sensor according to claim 8, characterized in that the additive is bovine serum albumin, glutaraldehyde, carbodiimide or a water-soluble polymer.

10. Sensor according to claims 2, 5 and 6, characterized in that the mixture (38) deposited on the collector of the measuring electrode (20) comprises between 1 and 2000 IU of glucose oxidase per mg of carbon powder and between 1 and 10000 μmol of mediator per mg of carbon powder.

11. Sensor according to claim 10, characterized in that the mixture (38) deposited on the collector of the measuring electrode (20) comprises between 10 and 300 IU of glucose oxidase per mg of carbon powder and between 10 and 300 μmol of mediator per mg of carbon powder.

12. Sensor according to claim 11, characterized in that the mixture (38) deposited on the collector of the measuring electrode (20) comprises about 100 IU of glucose oxidase per mg of carbon powder and about 50 μmol of mediator per mg of carbon powder.

**13.** Apparatus comprising a sensor according to any one of claims 1 to 12 and a device for processing the signal supplied by said sensor, said device comprising at least two electrical contacts adapted to be connected to at least two electrodes of said sensor, an ammeter and means for displaying the results.

$H_2 O_2$  $GOD_{(oxy)}$  Glucose

$O_2$  GOD $(H_2)$ (réd)  Gluconolactone

# Fig.1

$2e^-$  $H_2O_2$  $GOD_{(oxy)}$  Glucose

C  $O_2$  GOD $(H_2)$ (réd)  Gluconolactone

# Fig. 2

$H_2 O_2$

$O_2$

$GOD_{(oxy)}$    Glucose

Méd (réd)

$O_2$

$e^-$

Méd (oxy)

$GOD (H_2)$ (réd)

Gluconolactone

C

## Fig. 3

6   38   32   36   34

37

20

22

30

40   24   28   26

19

8   12   4    16

mg/dl

10   2   14

## Fig. 4

18

Fig. 5

Fig. 6

Fig.7

Fig.8

Fig. 9

Fig. 10

Fig.11

Fig.12

Fig. 13

Fig.14

Fig.15

Fig. 16